# EUROPEAN PATENT SPECIFICATION

(11) **EP 3 744 338 B1**
(45) Date of publication and mention of the grant of the patent: **01.10.2025**
(21) Application number: 19177491.8
(22) Date of filing: 30.05.2019
(51) Int. Cl.: A61K 36/15, A61P 35/00, A61P 3/10

(54) **MEDICAL USES OF RED PINE RESIN AND DOSAGE FORMS THEROF**
MEDIZINISCHE VERWENDUNG VON ROTKIEFERNHARZ UND DOSIERUNGSFORMEN DAVON
UTILISATIONS MÉDICALES DE RÉSINE DE PIN ROUGE ET LEURS FORMES POSOLOGIQUES

(43) Date of publication of application: 02.12.2020
(73) Proprietor: Tekdemir, Canseven, Sakarya (TR); Tekdemir, Ozan, Istanbul (TR); Keles, Ceren, Sakaraya (TR); Tekdemir, Irem, Istanbul (TR)
(72) Inventor: Tekdemir, Ismail, Adapazari (TR)
(74) Representative: Mutlu, Aydin

(56) References cited:
- WO-A1-01/91777
- DATABASE WPI Week 200053, Derwent World Patents Index; AN 2000-565806, XP002794453
- DATABASE WPI Week 200939, Derwent World Patents Index; AN 2009-K06241, XP002794397
- KIZILARSLAN ET AL: "Ethnobotanical uses of genus Pinus L. (Pinaceae) in Turkey", INDIAN JOURNAL OF TRADITIONAL KNOWLEDGE, NATIONAL INSTITUTE OF SCIENCE COMMUNICATION AND INFORMATION RESOURCES, NEW DELHI - INDIA, vol. 12, no. 2, 1 April 2013 (2013-04-01), pages 209 - 220, XP018029686
- FRANCOIS SIMARD ET AL: "Isolation and identification of cytotoxic compounds from the wood of Pinus resinosa", PHYTOTHERAPY RESEARCH, JOHN WILEY & SONS LTD. CHICHESTER, GB, vol. 22, no. 7, 1 January 2008 (2008-01-01), pages 919 - 922, XP002548831, ISSN: 0951-418X, [retrieved on 20080403], DOI: 10.1002/PTR.2416
- SATIL FATIH ET AL: "Ethnic uses of pine resin production from Pinusbrutia by native people on the Kazdag Mountain (Mt. Ida) in Western Turkey", INTERNATIONAL JOURNAL OF FOOD, AGRICULTURE & ENVIRONMENT, WFL, HELSINKI, FI, vol. 9, no. 3-4, Part 2, 30 June 2011 (2011-06-30), pages 1059 - 1063, XP009516154, ISSN: 1459-0255

## Description

### Technical Field

The present invention relates to red pine resin for oral use in treatment of type II diabetes and cancer.

More particularly, the invention relates to oral medical uses of red pine *(Pinus brutia)* resin comprising turpentine, which is derived from red pine (calabrian pine) trees that grow at Aegean and Mediterranean regions of Turkey.

### Background of the Invention

Red pine *(Pinus brutia)* is a species of pine native to the eastern Mediterranean region of Turkey. It is also known by several other names such as Turkish pine, Calabrian pine, East Mediterranean pine and Brutia pine. The species is mostly found in coastal areas of Aegean and Mediterranean regions, since it generally grows at low altitudes, mostly from sea level up to 600 meters. *Pinus brutia* is a medium-size tree, reaching 20-35 meters tall at the most. The bark is characterized as orange-red and thick. The leaves are in the form of needles and are in pairs. The red pine have stout, heavy and hard cones. The cones open slowly as they mature and release the seeds *(Satil, F., Selvi, S. & Polat, R. (2011). Ethnic uses of pine resin production from Pinus brutia by native people on the Kazdaǧ Mountain (Mt. Ida) in Western Turkey. Journal of Food, Agriculture & Environment. SCI. 1059-1063;* Gezer, A. 1986. The silviculture of Pinus brutia Ten. in Turkey. Ciheam 86(1):55-66*;* Frankis, M. 1999. Pinus brutia. Curtis's Botanical Magazine 16:173-184*).*

There are many uses of pine products related to their various benefits. WO 2008/004025 is concerned with nutritional or pharmaceutical compositions comprising extracts or concentrates of plants and the mixtures thereof belonging to *Pinus sp.* with specific reference to *Pinus pinea.*

The main product that is derived from red pine is pine honey or honeydew honey. Red pine is host to sap-sucking aphid species *Marchalina hellenica.* The presence of this insect results in secretion of excess sugar by red pine. This excess sugar, namely "honeydew", is then collected by honey bees and turned into a richly flavoured honey called pine/honeydew honey. WO 2015/097640 is related to a composition for the treatment of persistent cough which is also suitable also for paediatric use. The patent document also provides the preparation method of said composition. The composition of said patent document can contain bee honey and/or honeydew honey. Antioxidants, polysaccharides, resins and saponins are derived from the above-described sources. Also, an article titled *"Determination of the total phenolic, flavonoid and proline contents in Burkina Fasan honey, as well as their radical scavenging activity"* investigates the phenolic, flavonoid and proline contents as well as radical scavenging activity of 27 samples of honey, including two types of honeydew honeys *(*Meda, A., Lamien, C. E., Romito, M., Millogo, J., & Nacoulma, O. G. (2005). Determination of the total phenolic, flavonoid and proline contents in Burkina Fasan honey, as well as their radical scavenging activity. Food Chemistry, 91(3), 571-577*).*

In addition to honey, resin obtained from red pine is an important non-wood forest product. The topography and the distribution area of red pine allows it to be a suitable source of resin. Resin is secreted by the plant to heal wounds and kill insects and fungi. It also allows plant to eliminate excess metabolites *(*Hillis, W. 1987. The future of forest chemicals. Chinese Academy of Forestry, Chemistry and Industry of Forest Products 7(1):1-13*).* Pine resin (oleoresin) is traditionally obtained by chipping the bark of the tree. The pine resin is composed of acidic and neutral diterpenes, along with other volatile compounds *(Tümen, İ. and Reunanen, M. A. 2010. Comparative study on turpentine oils of oleoresins of Pinus sylvestris L. from three districts of Denizli. Rec. Nat. Prod. 4(4):224-229).* WO 2008/004025 is concerned with nutritional or pharmaceutical compositions comprising extracts or concentrates of plants and the mixtures thereof belonging to *Pinus sp.* with specific reference to *Pinus pinea.* The extracts have been screened and characterized for their activity in the treatment of increased bone resorption and bone formation, osteoporosis in particular. It is also stated in the document that pine trees produce a resin with a content of turpentine, which is known for its antiseptic, diuretic, rubefacient and vermifuge properties.

Turpentine (also known with the names of spirit of turpentine or oil of turpentine) is obtained in a fluid form by the distillation of resin. It is used in many areas of industry including medicine, pharmacy, food and cosmetics *(*Rezzi, S., Bighelli, A., Castola, V. and Casanova, J. 2005. Composition and chemical variability of the oleoresin of Pinus nigra subsp. Laricio from Corsica. Ind. Crop. Prod. 21:71-79). Therefore, turpentine is an economically and medically important product of pine species. It is beneficial for respiratory system, treating diseases of the mucous membranes and respiratory complaints such as coughs, colds, influenza and TB. WO 2017/142491 is related to an enhanced pomade having a combination which can be used in treatment of burns. Said pomade also has a pain killer activity. It is obtained by mixing certain amounts of olive oil, beeswax, turpentine, gunnuk gum, gum mastic, albies gum, chesmek, radix alkannae. *"Antioxidant and analgesic activities of turpentine of Pinus nigra Arn. subsp. pallsiana (Lamb.) Holmboe"* aims to examine possible antioxidant and analgesic activities of turpentine exudes from *Pinus nigra* Arn. subsp. *pallsiana* (Lamb.) Holmboe (TPN). Antioxidant activity is investigated in terms of total antioxidant activity, reducing power, superoxide anion radical scavenging, free radical scavenging, metal chelating and hydrogen peroxide scavenging activity. The results obtained indicated that TPN has a potential source of natural antioxidant. In addition to this, TPN is found to have a strong analgesic effect *(Gülçin, İ., Büyükokuroǧlu, M. E., Oktay, M., & Küfrevioġlu, Ö. İ. (2003). Antioxidant and analgesic activities of turpentine of Pinus nigra Arn. subsp. pallsiana (Lamb.) Holmboe. Journal of Ethnopharmacology, 86(1), 51-58).*

Diabetes, originally referred as Diabetes mellitus (DM), can be defined as a group of metabolic disorders. These disorders are characterized by high blood sugar levels over a prolonged period, which result from inability of pancreas to produce insulin or inability of the body to use the insulin efficiently. Since insulin is one of the most important anabolic hormones, defects of the insulin metabolism not only affect glucose metabolism of the tissues, but also have a negative impact on fat and protein metabolism *(*American Diabetes Association. Diagnosis and Classification of Diabetes Mellitus. Diabetes Care Jan 2010, 33 (Supplement 1) S62-S69*).* There is an article in the literature which investigates the effects of the pine oil on some biochemical parameters in muscle tissue of streptozotocin-induced type-1 diabetic rats. Said parameters are fatty acid composition, A, D, E and K vitamins, cholesterol and sterol levels. It was concluded that the pine oil has beneficial effects in decreasing some biochemical metabolic disorders caused by experimental diabetes in the muscle tissue *(Demir, E., Yilmaz Ö. Tip-1 Diyabet Olu* 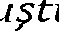 *turulan Stçanlarm Kas Dokusunda Bazi Biyokimyasal Deǧisikliklere Kar* 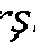 *i Çam Yaǧinin Etkisi. Marmara Fen Bilimleri Dergisi 2015, 4: 114-124).* KR 2018/0033781 relates to a preparation method of a food for preventing and alleviating diabetes. Said method comprises adding 1.5-2.5 wt% of pine needles to a mixture of natural ingredients.

Cancer is a medical term used for a group of diseases that are characterized with abnormal cell growth and have the potential to spread to other parts of the body through the blood and lymph systems. There are over 100 types of cancers that affect humans. These types are classified according to the type of cells they initially arise. US 7015248 relates to a novel use of abietic acid or derivatives thereof for inhibiting the growth of a cancer cell or treating a cancer, preferably, reducing the tumor size of the cancer. It is stated in the said document that abietic acid is one of the major ingredients of pine resin, and can be used in combination with a anticancer agent in order to improve the therapeutic effect of the said anticancer agent.

However, it is noted in prior art that medical use of pine resin doesn't go beyond traditional use and lacks of a certain dose schedule. The inventor has unexpectedly noted in several of experiences that patients receiving pine resins out of a certain dosage regime and with a turpentine content out of certain levels mostly fail in therapy due to either no therapeutic benefit or severe side effects such as headache. The latter is primarily caused by the efffect of pine resin on blood pressure of the patient which in turn causes hypertension. Furthermore, it is noted that not all resins obtained from pine trees would be beneficial for health in terms of the desired therapeutic effect and side effects.

In the light of the foregoing, it is clear that new compositions and doses of pine resin are necessary in the field of natural medical products. By this way, treatment of the common diseases disclosed herein can be achieved effectively without adverse effects.

### Summary of the Invention

The present invention addresses the above problems and provides a resin of red pine *(Pinus brutia)* for use orally in the treatment of type II diabetes mellitus and/or cancer with a dose from 500 mg to 12.000 mg wherein amount of turpentine in the resin ranges from 2,6 to 3,4 % (w/w), more preferably from 2,8 to 3,2 % by weight, and most preferably of about 3 % by weight

Each unit dose of the resin is arranged inbetween 500 mg and 8.000 mg, and more preferably inbetween 1.500 mg and 2.500 mg. Said doses are contemplated for use twice daily such that each dose is taken with about 12 hours of intervals. Duration of the treatment may vary between 3 months and 36 months.

Red pine resin according to the present invention is preferably in the form of an oral gel, capsule, tablet, buccal tablet, solution, suspension or syrup. More preferably it can be provided as a crude powder or in the form of a sublingual or chewable tablet

The cancer mentioned herein can be selected from the group consisting of skin cancer, stomach cancer, uterine cancer, breast cancer, pancreatic cancer, lung cancer or liver cancer.

In another aspect, the present invention provides resin of red pine *(Pinus brutia)* for use as identified above which is produced by a method comprising collecting of red pine resin after 6-18 months after secretion from the red pine trees, analyzing turpentine content, and adjusting amount of turpentine to the range of 2.6 to 3.4 % (w/w) in the overall resin. The resulting material can be brought into powder or pelletized form. The method may further comprise dividing of the resin material into unit doses ranging from 500 mg to 12.000 mg. The adjustment mentioned above may be carried out by way of evaporating turpentine under heating at a temperature, for instance, of 50 °C.

### Detailed Description of the Invention

The present invention provides resin obtained from red pine *(Pinus brutia)* for use in treatment of cancer or type II diabetes mellitus with a dose from 500 mg to 12.000 mg wherein amount of turpentine in the resin ranges from 2.6 to 3.4 % (w/w). In preferred embodiments, amount of turpentine ranges from 2.8 to 3.2 %, and most preferably is around 3.0 (w/w).

It has been unexpectedly discovered that red pine resin can be effective for the treatment of various cancers as well as Type II diabetes mellitus without or minimum of side effects or failing in therapy as far as it is used with the dose values and turpentine content as mentioned above. Red pine mentioned herein is a very specific one as explained hereinabove which belongs to the species of *Pinus brutia* grown in Aegean and Meditarrenean regions of Turkey.

When patients were given the red pine resin, the biological parameters that are defected because of type II diabetes were noted to be going to the normal levels. It was also noticed that cancer development slows down by way of treatment with red pine resin according to the present invention.

It was noted that amount of turpentine in the bulk mass of the red pine resin is important for obtaining the desired therapeutic effect and avoiding undesired side effects, for instance headache and increased blood pressure. Normally, red pine resin which is newly secreted from the trees contains about 10% by weight of turpentine which is not desirable in the context of the present invention. Resin with such a high amout of turpentine is noted to be detrimental in terms of the side effects. The inventor note that the resin needs to be collected during 6 to 18 months following it's leakage from the red pine trees. This period of time helps the ratio of volatile turpentine to be reduced and red pine resin to mature with the help of air, sun, rain, hot and cold weathers that the tree is exposed to. Eventually, it is desired to obtain a turpentine ratio below 3.4 % (w/w) in order to alleviate side effects. It is also desired to obtain a turpentine ratio over 2.8 % (w/w) for the desired therapeutic effect. Therefore, it is desirable to analyze the red pine resin after collection and adjust the amount of turpentine by way of a heating process.

Each dose of the resin is arranged inbetween 500 mg and 12.000 mg, more preferably 500 mg and 8000 mg, and most preferably between 1500 mg and 2500 mg. The doses according to the present invention can be administered once or twice in a day. If two separate doses are given, the interval between doses may be about 12 hours.

Optimum dose for each patient can be determined by dose titration accompanied with measuring of blood pressure, adrenalin secretion and adverse effects (i.e. headache). An optimal treatment may, for instance, start with a dose of 1500 mg (twice daily) by stepwise increase of the doses with 100 mg in each administration. After finding the optimum dose devoid of undesired effects, the dosage regime may continue for 3-36 months depending on improvement of the illness and symptoms.

The red pine resin according to the present invention is preferably administered orally to the patients. It can be consumed in its pure state, and taken orally in the form of a gel, capsule, tablet, buccal tablet, sublingual tablet, chewable tablet, solution, suspension or syrup.

Therefore, the present invention provides a red pine resin for use according to the present invention which is prepared by a method comprising the steps of:
- collecting red pine resin after 6-18 months after secretion from the trees,
- analyzing turpentine content,
- adjusting amount of turpentine to the range of 2.6 to 3.4 % (w/w) in the overall resin, and
- bringing the resin into powder or pelletized form.

The oral dosage form mentioned above can be suspended and/or solubilized in a liquid, or it can be provided in tablet form by compressing said powders or pellets.

In the context of the present invention, diabetes refers to Type I and Type II diabetes. The cancer to be treated can be selected from a group consisting of skin cancer, stomach cancer, uterine cancer, breast cancer, pancreatic cancer, lung cancer or liver cancer.

Further aspects and embodiments of the present invention can be contemplated from the following examples and appended claims.

### EXAMPLES

### A) Treatment of Patients having Type II Diabetes with Red Pine Resin

The experiments were conducted with numerous patients who were diagnosed with type II diabetes. Among these patients, data about three patients can be found in Table 1.

**Table 1. Type II Diabetic Patients' Age and Gender Data**

| **Patient** | **Age** | **Gender** |
|---|---|---|
| Patient 1 | 42 | Female |
| Patient 2 | 26 | Female |
| Patient 3 | 63 | Male |

The patients were observed both during the time they were given standard medication for type II diabetes and during the time they were using the red pine resin according to the present invention. It was shown that the red pine resin by itself can keep the blood values related to type II diabetes at normal levels. The data of three patients during treatment with standard medication and during treatment with red pine resin are given in Table 2. During treatment with red pine resin the patients were only given 2000 mg of red pine resin two times a day with 12 hours of intervals. Total daily dosage of the red pine resin was 4000 mg daily. The treatment was not accompanied with standard medication for type II diabetes.

**Table 2. Blood Levels of Patients During Treatment with Standard Medication and During Treatment with Red Pine Resin**

| | | **HbA1C (%)** | **Glucose (preprandial) (mg/dL)** | **Insulin (preprandial) (µIU/mL)** | **Urea (mg/dL)** | **Total Cholesterol (mg/dL)** | **HDL Cholesterol (mg/dL)** | **LDL Cholesterol (mg/dL)** | **Triglyceride (mg/dL)** | **AST (IU/L)** | **ALT (IU/L)** |
|---|---|---|---|---|---|---|---|---|---|---|---|
| **Reference Range** | | 4,8-5,9 | 70-100 | 2,6-25 | 15-43 | 125-200 | 40-80 | 60-130 | 50-150 | 10-32 | 6-33 |
| **Patient 1** | **Treatment with Standard Medication** | 5,2 | 81,3 | 16,17 | 16,27 | 214,3 | 43,9 | 161,1 | 121,7 | 15,6 | 15,9 |
| | **Treatment with Red Pine Resin** | 5,3 | 84 | - | 23 | 263 | 45 | 199 | 95 | 16 | 14 |
| **Patient 2** | **Treatment with Standard Medication** | 5,08 | 88,5 | 17,01 | 18,84 | 185,9 | 58,6 | 105,54 | 108,8 | 14,5 | 10,4 |
| | **Treatment with Red Pine Resin** | - | 80 | 18 | - | 200 | 62 | 116,2 | 109 | 12 | 9 |
| **Patient 3** | **Treatment with Standard Medication** | 10,5 | 199 | - | 38 | 266 | 49,2 | 201 | 93 | 13 | 15 |
| | **Treatment with Red Pine Resin** | 8,8 | 235 | - | 32 | 202 | 52,4 | 133 | 71 | 14 | 10 |

As it can be seen from Table 2, treatment with only red resin pine enabled similar blood values to be achieved, compared to treatment with standard medication. Moreover, type II diabetes related complaints of the patients were relieved. Said standard medication was Glifor 1000 mg for Patient 1 (once a day) and Matofin 1000 mg for Patient 2 (2 times a day).

### B) Treatment of Patients with Cancer with Red Pine Resin

The experiments were conducted with three patients (Patient 4, Patient 5 and Patient 6) who were diagnosed with skin cancer, stomach cancer and liver cancer, respectively. Data relating to age and gender of these patients are given in Table 3.

**Table 3. Cancer Patients' Age and Gender Data**

| **Patient** | **Age** | **Gender** |
|---|---|---|
| Patient 4 | 54 | Male |
| Patient 5 | 48 | Male |
| Patient 6 | 70 | Female |

Among these patients, Patient 4 was diagnosed with skin cancer in 2013, Patient 5 was diagnosed with stomach cancer in 2006, and Patient 6 was diagnosed with liver cancer in 2018. The patients were given 2.500 mg of red pine resin two times a day. Total daily dosage of the red pine resin was 5.000 mg. All patients reported relief in symptoms of the diseases and clinical improvement with regards to the tumours.

For Patient 4, HDL levels were found to be high throuhout the course of the disease. HDL levels are known to be closely related to cancer development. When given the red pine resin according to the present invention, HDL levels of patient decreased to reference range. The data is shown in Table 4. Moreover, the occurence and the size of lesions of this patient were surprisingly reduced. The patient started to use red pine resin on 02.06.2018.

**Table 4. Cholesterol Data for Patient 4**

| | | |
|---|---|---|
| **Patient 4** | **HDL Level on 20.02.2018 (mg/dL)** | **HDL Level on 05.09.2018 (mg/dL)** |
| | 64 | 44 |

For Patient 5, the overall condition of the patient was improved with the treatment with red pine resin.

For Patient 6, alfa-feto protein (AFP) levels were found to be high throughout the course of the disease. As it is known by persons skilled in the art, alfa-feto protein is commonly used for diagnosis and follow-up of various types of cancer. It was shown that patient's AFP levels drastically reduced when given the red pine resin of the present invention. The data regarding the AFP levels of Patient 6 is given in Table 5. The patient started to use red pine resin on 27.12.2018.

**Table 5. AFP Data for Patient 6**

| | **Alfa-feto Protein (AFP) Levels** | | | | |
|---|---|---|---|---|---|
| **Patient 6** | **14.11.2018** | **17.12.2018** | **26.12.2018** | **10.01.2019** | **19.02.2019** |
| | 2874 | 7136 | 8175 | 2921 | 35,33 |

In addition to this, the metastase level of Patient 6 regressed with the treatment with red pine resin.

## Claims

1. Resin of red pine *(Pinus brutia)* for use orally in the treatment of type II diabetes mellitus and/or cancer with a dose from 500 mg to 12.000 mg wherein amount of turpentine in the resin ranges from 2.6 to 3.4 % (w/w).

2. Red pine resin for use according to claim 1, wherein said red pine resin comprises 2.8% to 3.2% turpentine by weight

3. Red pine resin for use according to claim 2, wherein said red pine resin comprises 3% of turpentine by weight.

4. Red pine resin for use according to claim 1, wherein each dose of the resin is inbetween 500 mg and 12.000 mg.

5. Red pine resin for use according to claim 4, wherein each dose of the resin is inbetween 500 mg and 8.000 mg.

6. Red pine resin for use according to claim 4, wherein each dose of the resin is inbetween 1.500 mg and 2.500 mg.

7. Red pine resin for use according to claim 1, wherein the dose of resin is for use twice daily such that each dose is taken with 12 hours of intervals.

8. Red pine resin for use according to any one of the preceding claims, wherein the duration of the treatment is between 3 months and 36 months.

9. Red pine resin for use according to claim 1, wherein said red pine resin is in the form of an oral gel, capsule, tablet, buccal tablet, solution, suspension or syrup.

10. Red pine resin for use according to claim 9, wherein said red pine resin is in the form of a sublingual or chewable tablet.

11. Red pine resin for use according to claim 1, wherein said cancer is selected from a group consisting of skin cancer, stomach cancer, uterine cancer, breast cancer, pancreatic cancer, lung cancer or liver cancer.

12. Red pine resin for use according to claim 1, wherein the red pine resin *(Pinus brutia)* is prepared by a method comprising the steps of:
- collecting red pine resin after 6-18 months after secretion from the trees,
- analyzing turpentine content,
- adjusting amount of turpentine by evaporating it under heating to the range of 2.6 to 3.4 % (w/w) in the overall resin,
- bringing the resin into powder or pelletized form, and
- dividing the resin material into unit doses ranging from 500 mg to 12.000 mg.

13. Red pine resin for use according to claim 12 wherein heating of turpentine is carried out at 50 °C.

## Patentansprüche

1. Harz aus Rotkiefer *(Pinus brutia)* zur oralen Verwendung bei der Behandlung von Diabetes mellitus Typ II und/oder Krebs mit einer Dosis von 500 mg bis 12000 mg, wobei die Menge an Terpentin in dem Harz im Bereich von 2,6 bis 3,4 % (Gew./Gew.) liegt.

2. Rotkieferharz zur Verwendung gemäß Anspruch 1, wobei das Rotkieferharz 2,8 bis 3,2 Gew.-% Terpentin enthält.

3. Rotkieferharz zur Verwendung gemäß Anspruch 2, wobei das Rotkieferharz 3 Gew.-% Terpentin enthält.

4. Rotkieferharz zur Verwendung gemäß Anspruch 1, wobei jede Dosis des Harzes zwischen 500 mg und 12000 mg liegt.

5. Rotkieferharz zur Verwendung gemäß Anspruch 4, wobei jede Dosis des Harzes zwischen 500 mg und 8000 mg liegt.

6. Rotkieferharz zur Verwendung gemäß Anspruch 4, wobei jede Dosis des Harzes zwischen 1500 mg und 2500 mg liegt.

7. Rotkieferharz zur Verwendung gemäß Anspruch 1, wobei die Dosis des Harzes zur zweimaligen täglichen Einnahme vorgesehen ist, so dass jede Dosis in einem Abstand von 12 Stunden eingenommen wird.

8. Rotkieferharz zur Verwendung gemäß irgendeinem der vorstehenden Ansprüche, wobei die Dauer der Behandlung zwischen 3 Monaten und 36 Monaten liegt.

9. Rotkieferharz zur Verwendung gemäß Anspruch 1, wobei das Rotkieferharz in Form eines oralen Gels, einer Kapsel, einer Tablette, einer bukkalen Tablette, einer Lösung, einer Suspension oder eines Sirups vorliegt.

10. Rotkieferharz zur Verwendung gemäß Anspruch 9, wobei das Rotkieferharz in Form einer sublingualen oder kaubaren Tablette vorliegt.

11. Rotkieferharz zur Verwendung gemäß Anspruch 1, wobei der Krebs ausgewählt ist aus einer Gruppe, bestehend aus Hautkrebs, Magenkrebs, Gebärmutterkrebs, Brustkrebs, Bauchspeicheldrüsenkrebs, Lungenkrebs oder Leberkrebs.

12. Rotkieferharz zur Verwendung gemäß Anspruch 1, wobei das Rotkieferharz *(Pinus brutia)* durch ein Verfahren hergestellt wird, das die folgenden Schritte umfasst:
- Sammeln des Rotkieferharzes nach 6-18 Monaten nach der Absonderung von den Bäumen,
- Analysieren des Terpentingehalts,
- Einstellen des Terpentingehalts durch Verdampfen unter Erhitzen auf den Bereich von 2,6 bis 3,4 % (Gew./Gew.) im Gesamtharz,
- Bringen des Harzes in Pulver- oder Pelletform, und
- Aufteilen des Harzmaterials in Einheitsdosen im Bereich von 500 mg bis 12000 mg.

13. Rotkieferharz zur Verwendung gemäß Anspruch 12, wobei das Erhitzen des Terpentins bei 50 °C erfolgt.

## Revendications

1. Résine de pin de Turquie (Pinus brutia) pour utilisation par voie orale dans le traitement d'un diabète sucré de type II et/ou d'un cancer avec une dose de 500 mg à 12000 mg, dans laquelle résine la proportion d'essence de térébenthine vaut de 2,6 à 3,4 % pds/pds.

2. Résine de pin de Turquie pour utilisation conforme à la revendication 1, laquelle résine de pin de Turquie comprend de 2,8 à 3,2 % en poids d'essence de térébenthine.

3. Résine de pin de Turquie pour utilisation conforme à la revendication 2, laquelle résine de pin de Turquie comprend 3 % en poids d'essence de térébenthine.

4. Résine de pin de Turquie pour utilisation conforme à la revendication 1, de laquelle résine chaque dose vaut entre 500 mg et 12000 mg.

5. Résine de pin de Turquie pour utilisation conforme à la revendication 4, de laquelle résine chaque dose vaut entre 500 mg et 8000 mg.

6. Résine de pin de Turquie pour utilisation conforme à la revendication 4, de laquelle résine chaque dose vaut entre 1500 mg et 2500 mg.

7. Résine de pin de Turquie pour utilisation conforme à la revendication 1, de laquelle résine la dose est conçue pour être utilisée deux fois par jour de telle sorte que les doses soient prises chacune à 12 heures d'intervalle.

8. Résine de pin de Turquie pour utilisation conforme à l'une des revendications précédentes, avec laquelle la durée du traitement se situe entre 3 mois et 36 mois.

9. Résine de pin de Turquie pour utilisation conforme à la revendication 1, laquelle résine de pin de Turquie se présente sous la forme d'un gel oral, d'une capsule, d'un comprimé, d'un comprimé buccal, d'une solution, d'une suspension ou d'un sirop.

10. Résine de pin de Turquie pour utilisation conforme à la revendication 9, laquelle résine de pin de Turquie se présente sous la forme d'un comprimé sublingual ou à mâcher.

11. Résine de pin de Turquie pour utilisation conforme à la revendication 1, pour laquelle ledit cancer est choisi dans l'ensemble constitué par un cancer de la peau, un cancer de l'estomac, un cancer de l'utérus, un cancer du sein, un cancer du pancréas, un cancer du poumon et un cancer du foie.

12. Résine de pin de Turquie pour utilisation conforme à la revendication 1, laquelle résine de pin de Turquie (Pinus brutia) est préparée selon un procédé comportant les étapes suivantes :
- recueillir de la résine de pin de Turquie après 6 à 18 mois de sécrétion des arbres,
- en déterminer, par analyse, la teneur en essence de térébenthine,
- en ajuster la teneur en essence de térébenthine, par évaporation de celle-ci par chauffe, dans l'intervalle allant de 2,6 à 3,4 % pds/pds dans toute la résine,
- mettre la résine sous forme de poudre ou de pastilles,
- et diviser la résine en doses unitaires dont le poids vaut de 500 mg à 12000 mg.

13. Résine de pin de Turquie pour utilisation conforme à la revendication 12, pour laquelle la chauffe de l'essence de térébenthine est effectuée à 50 °C.
